# EUROPEAN PATENT SPECIFICATION

(11) **EP 0 939 622 B1**
(45) Date of publication and mention of the grant of the patent: **05.03.2003**
(21) Application number: 97909449.7
(22) Date of filing: 17.10.1997
(51) Int. Cl.: A61K 9/16, A61K 9/50, A61K 49/00

(54) **POROUS MICROCAPSULES AND THEIR USE AS THERAPEUTIC AND DIAGNOSTIC VEHICLES**
PORÖSE MIKROKAPSELN UND DEREN VERWENDUNG ALS TRÄGER FÜR THERAPEUTIKA UNDDIAGNOSTIKA
MICROCAPSULES POREUSES ET LEUR UTILISATION EN TANT QUE VEHICULES THERAPEUTIQUES ET DIAGNOSTIQUES

(30) Priority: 19.10.1996 GB 9621825
(43) Date of publication of application: 08.09.1999
(73) Proprietor: Elan Drug Delivery Limited, Nottingham, NG11 6JS (GB)
(72) Inventor: TURNBULL, Graham John, Cambridge CB2 5HP (GB); OSBORNE, Nicholas David, Quadrant Healthcare (UK), Ruddington, Nottingham NG11 6JS (GB)
(74) Representative: Perry, Robert Edward
(86) International application number: GB9702875
(87) International publication number: WO98017257

(56) References cited:
- EP-A- 0 076 515
- EP-A- 0 306 236
- EP-A- 0 466 986
- WO-A-91/04732
- WO-A-93/00050
- WO-A-93/07862
- WO-A-96/09814
- WO-A-96/18388
- US-A- 5 008 116
- US-A- 5 069 936
- DATABASE WPI Week 8849 Derwent Publications Ltd., London, GB; AN 88-348733 XP002053077 & JP 63 258 640 A (LION CORP) , 26 October 1988
- DATABASE WPI Week 8939 Derwent Publications Ltd., London, GB; AN 89-282482 XP002053078 & JP 01 207 133 A (MATSUMOTO YUSHI SEIYAKU KK) , 21 August 1989

## Description

### Field of the Invention

This invention relates to porous microparticles and to their use as therapeutic and diagnostic vehicles.

### Background of the Invention

WO-A-9618388 discloses spherical microparticles, 0.1-50 µm in diameter, of cross-linked material, the microparticles being hydrophilic and capable of reconstitution in water to give a mono-dispersed suspension. The microparticles additionally comprise a physiologically or diagnostically-active agent linked directly or indirectly to the microparticles *via* free functional groups thereon.

Such microparticles can be prepared as a result of the discovery that, while microparticles having very desirable physical characteristics can be obtained by controlled spray-drying techniques, those techniques do not substantially affect functional groups. Thus, despite the heating etc. used in spray-drying, a proteinaceous material such as human serum albumin (HSA) retains functional groups such as OH, COOH, NH₂ and SH which, after cross-linking of the particles, are available for bonding to active agents. The bonded materials are useful for therapeutic or diagnostic purposes, on account of the controlled particle size that can be obtained for the particles in the spray-drying procedure. Thus, for example, microparticles of a defined size can be used for the delivery of an appropriate drug, by means of a powder inhaler, to the alveoli.

A problem with this technology, for certain active materials, is that the amount that can be bonded to the particles is low. Further, cross-linking of the microparticles may reduce the number of available bonding sites. This problem may be relatively unimportant if the agent is a highly potent drug, but even then the effect of a drug may be masked to an undesirable effect by the microparticles acting as its carrier to the site of action. It is also possible that a predominant carrier may have a toxic effect, and/or may undesirably dictate drug release characteristics.

EP-A-0306236, EP-A-0466986, US-A-5008116, WO-A-9104732, WO-A-9300050 and WO-A-9307862 disclose various types of generally solid porous polymeric particles containing active agent retained in the pores. The agent may thus be subject, for example, to controlled release.

US-A-5069936 discloses cross-linked protein microspheres with controlled porosity, for a similar purpose. A biological agent is held on the surface and in the pores, to relative extents depending on the method of manufacture, and with a view to providing a desired degree of protection/presentation of the agent, in use.

### Summary of the Invention

The present invention is based on the utility of porous hollow microcapsules, and for a purpose different from controlled release. In particular, it has been discovered that the loading of drugs on microcapsules, of the type that can be obtained by spray-drying, can be greatly enhanced. The loading can be increased by a factor of at least 2, 3 or more, relative to the level of loading that is possible in the absence of any modification according to the present invention.

This and other desirable effects are achieved by rendering the walls of suitable microcapsules porous. The pores provide additional surface area, to which a physiologically or diagnostically-active agent can be chemically or physically linked, in addition to surface binding. The porosity may also be used as a means to introduce the agent into the microcapsules. It may also enhance biodegradability.

### Description of the Invention

In general, products of the invention may be prepared by the steps of producing the microcapsules, fixing/crosslinking them (if necessary or desired), rendering the microcapsules porous, and then introducing the added agent. The agent may be chemically or physically linked to, trapped in, or otherwise associated with, the pores in the walls of the microcapsules

The microcapsules themselves are suitably prepared by spray-drying, e.g. using the materials and techniques described, for example, in WO-A-9218164, WO-A-9609814 and WO-A-9618388. These publications also describe desirable sizes and size distributions. WO-A-9609814 and WO-A-9618388 (incorporated herein by reference) also describe agents that can be coupled to the microparticles, and various means for doing that. Cytotoxic agents such as methotrexate, cisplatin and doxorubicin are examples of such compounds that can be used in accordance with this invention.

Rendering the microcapsules porous can markedly increase their surface area, provided that the pores are of sufficient size to ensure wetting of the additional surface area. Further, the thicker the walls of the microcapsules, the more opportunity exists for providing advantages such as more bound drug, and fewer microcapsules per dose. Also, porous walls should increase the biodegradation rate *in vivo,* by allowing extracellular and intracellular enzymes access to a larger surface area for preliminary breakdown. In turn, that can accelerate overall breakdown and release of bound drug.

One way of rendering spray-dried microcapsules more porous involves spray-drying the wall-forming material with an additional component that can subsequently be removed from the walls, e.g. by treating the formed microcapsules with a solvent for that component. Thus, for example, a solution or suspension of a sugar such as lactose or a salt such as calcium carbonate or magnesium carbonate in HSA (used herein merely as an illustrative wall-forming material) can be spray-dried to yield microcapsules which, after heat or chemical fixation, can be treated in an aqueous medium to remove the sugar or salt. Depending on the particle size and on the loading of carbonate suspension, the porosity/pore size is controllable.

Likewise, a porous wall may be produced by co-spraying HSA with calcium alginate, heat-stabilising the microcapsules and, in an aqueous medium, removing the alginate by Na or EDTA.

To avoid exposing unstabilised microcapsules to an aqueous phase, there is the alternative of co-spraying HSA with, say, sodium benzoate, and suspending the resulting microcapsules in ethanol to remove the benzoate. This approach may also avoid the need to stabilise the microcapsules before removal of the temporary component. Co-spraying HSA and a lipid or wax (chosen for pharmaceutical acceptability), and removing the lipid in a pharmaceutically-acceptable organic solvent, also avoids the need to fix the capsule before removing the temporary component.

Porosity may also be introduced by chemical or physical treatment of intact microcapsules, fixed or unfixed. A suitable physical process comprises high energy ultrasound exposure of microcapsules suspended in a concentrated drug solution. This results in passage of the drug in solution into the central cavity. Subsequent removal of the water by, for example, lyophilisation should leave the drug within the microcapsule.

Ultrasound treatment of the microcapsules in a nonaqueous solvent, or followed by rapid transfer to a solvent, may also increase greatly the concentration of the drug in the medium, and hence the drug load entering the microcapsule. In such a case, the solvent should be pharmaceutically-acceptable, such as ethanol. Other pharmaceutically-acceptable solvents for the drug (or vehicle for suspended drug) may include lipids, particularly natural dietary components and/or naturally circulating lipids such as palmitic acid. Certain waxes with low melting point might also be acceptable and effective as carriers for dissolved or suspended drug. Retention of a vehicle such as palmitic acid within the microcapsule may be used to affect the *in vivo* drug release rate. In place of removing the vehicle by lyophilisation etc, a filtration or centrifugation step may be used, followed by a rapid solvent wash.

Microcapsules may be loaded with bound drug and then filled with unbound drug, to increase the total drug load. The half-life for *in vivo* release may be different for bound and unbound drug, providing a burst of released (unbound) drug, followed by slower release of bound drug.

The following Example illustrates the invention.

### Example

HSA was spray-dried with 0% (control), 10% and 25% lactose. The microparticles containing lactose were then stabilised by the use of heat (H) or chemical cross-linking (X) , half of each type were washed (W) , and all were loaded with fluorescein isothiocyanate (FITC). In the following Table, therefore, "Lac 25 X W" refers to washed, chemically cross-linked microparticles containing 25% lactose. In each case, the results of 2 different runs are provided under "spec. Reading".

The data clearly indicate that increased loading is achieved by using lactose, especially if washing is used as an intentional means of removing it. In all cases, loading is increased by a factor of more than 2, with respect to the control.

| Sample | Spec. reading (µm/ml) | Mean Number Of FITC Moles Bound |
|---|---|---|
| Lac 25 X W | 0.9126/1.3406 | 10.33 |
| Lac 25 X | 1.0813/0.8115 | 8.68 |
| Lac 25 H W | 0.3508/0.4413 | 3.63 |
| Lac 25 H | 0.3657/0.8008 | 3.51 |
| Lac 10 X W | 0.4357/0.3858 | 3.77 |
| Lac 10 X | 0.3047/0.2178 | 2.39 |
| Lac 10 H W | 0.0884/0.0940 | 0.84 |
| Lac 10 H | 0.0804/0.1661 | 0.75 |
| HSA (control) | 0.0440/0.0401 | 0.39 |

## Claims

1. Microcapsules each consisting of a wall defining a hollow, empty core, **characterised in that** the wall is porous.

2. Microcapsules according to claim 1, obtainable by co-spray-drying a wall-forming material and a material that can be removed from the capsule walls, and removing said material.

3. Microcapsules each comprising a porous wall defining a hollow core, and which additionally have an associated physiologically or diagnostically-active component, linked to the pores in the walls of the microcapsules.

4. Microcapsules according to claim 3, wherein the loading of the active component is a factor of at least two times that obtainable for the same size of non-porous microcapsules.

5. Microcapsules according to claim 4, wherein the factor is at least 3 times.

6. Microcapsules according to any of claims 3 to 5, for use in therapy or diagnosis.

7. Microcapsules according to any preceding claim, which are cross-linked.

8. Microcapsules according to any preceding claim, 0.1 to 50 µm in size.

## Patentansprüche

1. Mikrokapseln, von denen jede aus einer Wand besteht, die einen hohlen, leeren Kern begrenzt, **dadurch gekennzeichnet, daß** die Wand porös ist.

2. Mikrokapseln nach Anspruch 1, erhältlich durch gleichzeitiges Sprühtrocknen eines wandbildenden Materials und eines Materials, das von den Kapselwänden entfernt werden kann, und Entfernen des Materials.

3. Mikrokapseln, von denen jede eine poröse Wand umfaßt, die einen hohlen Kern begrenzt, und die zusätzlich eine damit verbundene physiologisch oder diagnostisch aktive Komponente aufweisen, die an die Poren in den Wänden der Mikrokapseln gebunden ist.

4. Mikrokapseln nach Anspruch 3, bei welchen die Beladung mit der aktiven Komponente mit einem Faktor von wenigstens zwei mal mehr erfolgt, als sie erzielbar ist für die gleiche Größe nicht poröser Mikrokapseln.

5. Mikrokapseln nach Anspruch 4, bei welchen der Faktor wenigstens 3 ist.

6. Mikrokapseln nach einem der Ansprüche 3 bis 5 zur Verwendung in der Therapie oder Diagnostik.

7. Mikrokapseln nach einem der vorstehenden Ansprüche, die vernetzt sind.

8. Mikrokapseln nach einem der vorstehenden Ansprüche mit einer Größe von 0,1 bis 50 µm.

## Revendications

1. Microcapsules formées chacune d'une paroi définissant un noyau creux, vide, **caractérisées en ce que** la paroi est poreuse.

2. Microcapsules selon la revendication 1, pouvant être obtenues par séchage par co-pulvérisation d'un matériau de formation d'une paroi, et d'un matériau qui peut être éliminé des parois de la capsule, et élimination du dit matériau.

3. Microcapsules comprenant chacune une paroi poreuse définissant un noyau creux, et qui possèdent de plus un composant associé, actif du point de vue physiologique ou de diagnostic, lié aux pores dans les parois des microcapsules.

4. Microcapsules selon la revendication 3, dans lesquelles la charge du composant actif est un facteur valant au moins deux fois celui qui peut être obtenu pour la même taille de microcapsules non poreuses.

5. Microcapsules selon la revendication 4, dans lesquelles le facteur vaut au moins 3 fois.

6. Microcapsules selon l'une quelconque des revendications 3 à 5, à utiliser pur la thérapie ou le diagnostic.

7. Microcapsules selon l'une quelconque des revendications précédentes, qui sont réticulées.

8. Microcapsules selon l'une quelconque des revendications précédentes, d'une taille de 0,1 à 50 µm.
